# EUROPEAN PATENT APPLICATION

(11) **EP 1 172 067 A1**
(43) Date of publication of application: **16.01.2002**
(21) Application number: 00305913.6
(22) Date of filing: 12.07.2000
(51) Int. Cl.: A61B 5/16

(54) **Intelligence testing and training system**

(71) Applicant: Well Being Corporation, Uji-shi, Kyoto (JP)
(72) Inventor: Takagi, Hitoshi, Urawa-shi, Saitama (JP)
(74) Representative: Jacob, Reuben Ellis

(57) **Abstract**

An intelligence test and recovery training system, comprising a storage means (11) for storing a test program, and a field image that consists of a test field corresponding to the test program and a guide pattern selectively explicitly shown in the field, a field image generating means (12) for reading the field image from the storage means and generating image signals, a display means (3) having a screen frame (2) for displaying the field image according to the image signals, pointing means (4) for pointing a position where a test subject makes an entry of a dot or line in the displayed test field on the basis of a system planned in a test program therefor, an entry position detecting and storing means (13) for driving the display means (3) so as to detect the positions of dots or lines entered by the test subject and selectively display them in the field and store the positions and locus of the dots or lines together with the lapse of time, and a controlling and operating means (14) for evaluating the degree of intelligence or the state of intelligence recovery by statistically computing or analyzing firstly the relationship between the positions and locus of the dots or lines entered by said test subject and the lapse of time, and secondly the distribution, or by computing the degree of agreement between the entry positions and the guide pattern, or computing a time lag and positional shift.

## Description

The present invention relates to an intelligence testing and recovery training system.

In recent years, an increase in the elderly people bracket has presented various problems about supporting the living of these people. Particularly, the problem of intelligence lowering, which is said to occur with aging, influences dignity of man. If a symptom of intelligence lowering is observed upon test, besides providing corresponding care or support, which is a matter of course, it is desired to provide appropriate intelligence recovery training so as to at least prevent aggravation of the disease.

As for tests for intelligence lowering of man, medical oral examination and medical examination using test paper have been common.

According to these, much time is taken because of a time lag between personal appearance for a test before a doctor and delivery of examination results, and the cost involved therein is not negligible. Therefore, such intelligent testing system can hardly cope with an increase in the elderly people population and is not necessarily satisfactory. The same may be said of intelligence recovery training, and under the present conditions no tool designed to readily perform such training has been proposed as yet.

The present invention, which has been accomplished with the above points in mind, has for its object the provision of an intelligence testing device and an intelligence recovery training system, designed to readily perform intelligence test and intelligence recovery training and let the test results be known at once.

According to the invention, the above object is achieved by an intelligence test and recovery training system, comprising;
(a) a test program and field image storing means for storing at least one test program, and a field image that consists of a test field constructed according to said program and a guide pattern used for recognition, chase, replicating or the like and that is selectively explicitly shown in said field,
(b) a field image generating means for reading the field image from said storage means according to the program and generating image signals,
(c) a field image displaying means having a screen frame for receiving said image signals and displaying the field image,
(d) a pointing means that, in order to allow a testee or subject to make an entry of a dot or line in the test field displayed in said screen frame, on the basis of a random dotting system or a system in accord with a guide pattern explicitly shown in the field, which is planned in a test program therefor, is used to point the entry position therefor,
(e) an entry position generating and storing means for driving said display means so as to detect the positions and locus of the dots or lines entered by said testee and selectively display the same in said field according to the program and storing the positions and locus of the dots or lines together with the lapse of time, and
(f) a controlling and operating means for evaluating the degree of intelligence or the state of intelligence recovery by statistically calculating and analyzing the distribution of the dots, and the relationship between the positions and locus of the dots or lines entered by said testee and the lapse of time, or by computing the degree of recognition of the guide pattern or time lag and positional shift.
   In the present invention, the controlling and operating means is capable of causing said display means to display the testee's evaluated degree of intelligence or his evaluated state of intelligence recovery. Therefore, the display means can be divided into two parts, respectively consisting of a field entry section and a test result notification section. The field entry section of the display means comprises a liquid crystal display means for displaying a plurality of squares arranged in matrix form. The controlling and operating means is capable of performing the following operations according to a test program; (1) computing the test results from the number of squares entry-indicated by the indicating means and the positions of such squares and from the time spent for pointing, (2) computing the test results by causing the liquid crystal display means to display a random-moving target as an image in the test field and using the time or distance upon the target having been successfully indicated by the indicating means, or using the time or distance passed upon the target having been unsuccessfully pointed by the pointing means, and (3) computing the test results by causing the liquid crystal display means to display a plurality of squares as an image in the test field and also display for a given time a target to be chased in at least one of the squares randomly successively selected from said displayed squares and using the number of targets pointed by the pointing means during the display of the target and the number of targets displayed during test.

Further, it may be constructed to perform the following operations depending on the test program; (4) computing the test results by causing the liquid crystal display means to display a plurality of squares as an image in the test field, making a comparison between the order of the squares pointed by the pointing means and the preset order of routing the squares for testee's indication (which order is set by giving an instructors pre-explanation, for example, "move along a diagonal line from the upper right to the lower left", or by displaying in advance an order route for a short time) and using the time taken to complete the indication of the squares in the preset order of arrangement of the squares to be pointed or the number of times of erroneous indication, (5) computing the test results by causing the liquid crystal display means to display a plurality of different figures as a recognition pattern image in the test field and using pointing percentage of particular figures by the pointing means, and (6) computing the test results by causing the liquid crystal display means to display an original figure in the test field and using the difference between the pointed locus for replicating the original figure by the pointing means and the displayed original figure.

In the intelligence test system of the invention, the displaying means displays the image of a test object by image signals fed from the field image generating means. A testee points predetermined places in the test field displayed in the display means, on the screen through the pointing means. The controlling and computing means that carries out computations according to the test program detects the pointed positions provided by said pointing means and computes the test results, that is, the degree of intelligence, etc., on the basis of this detection result and the test program. The notifying means notifies the testee of the test results provided by the processing means.
The invention will now be described in more detail on the basis of preferred embodiments shown in the drawings.
Fig. 1 is a view showing a roughly grasped arrangement of a preferred embodiment of the present invention;
Fig. 2 is a view in which, in said embodiment, a pen type pointing means for use by a testee in making an entry in a test field is shown with a background of the field when a typical test program is to be executed;
Fig. 3 is a view showing, in said embodiment, a state in which in order to make an entry in the test field, the field itself is displayed on a finger touch screen;
Fig. 4 is a view in which, in said embodiment, a laser pointer type pointing means for use by a testee in making an entry in the test field is shown with a background of the field when the typical test program is to be executed;
Fig. 5 is a view in which, in said embodiment, a mouse type pointing means for use by a testee in making an entry in the test field is shown with a background of the field when the typical test program is to be executed;
Fig. 6 is a view in which, in said embodiment, a head stick type pointing means for use by a testee in making an entry in the test field is shown with a background of the field when the typical test program is to be executed;
Fig. 7 is a view in which, in said embodiment, a mouse stick type pointing means for use by a testee in making an entry in the test field is shown with a background of the field when the typical test program is to be executed;
Fig. 8 is a view in which, in said embodiment, a head master type pointing means for use by a testee in making an entry in the test field is shown with a background of the field when the typical test program is to be executed;
Fig. 9 is a block diagram showing the arrangement of a preferred embodiment of the invention;
Fig. 10 is a view showing, in said embodiment, the relationship between a field and pointing means for the field when a different test program is to be executed;
Fig. 11 is a view showing, in said embodiment, the relationship between a field and an indication means for the field when another test program is to be executed;
Fig. 12 is a view showing, in said embodiment, the relationship between a field and pointing means for the field when still another test program is to be executed;
Fig. 13 is a view showing, in said embodiment, the relationship between a field and pointing means for the field when still another test program is to be executed; and
Fig. 14 is a view showing, in said embodiment, the relationship between a field and pointing means for the field when still another test program is to be executed.

Preferred embodiments of the invention shown in the drawings will now be described in more detail.

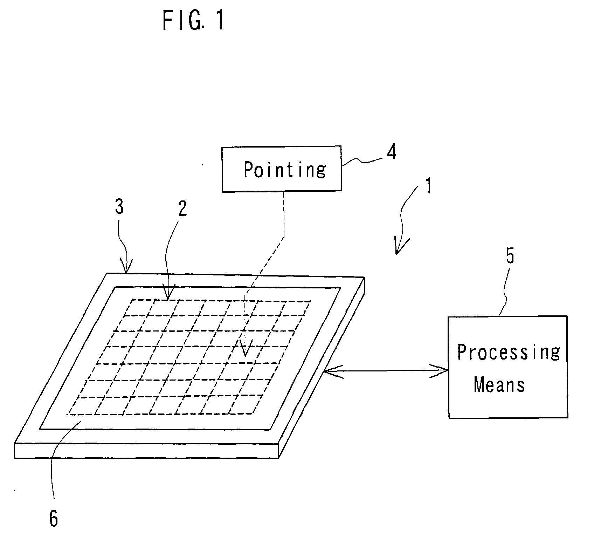

An intelligence testing and recovery training system 1 according to the example shown in Fig. 1 comprises a display means 3, such as liquid crystal, for displaying a test field image on a screen 2 according to an image signal fed in, pointing means 4 whereby in order for a testee to put a dot or make an entry of a line in a manner taught by an instructor or the like according to a test program, a predetermined location in the test field displayed on the screen 2 of the display means 3 is indicated onto the screen 2 of the liquid crystal display means 3, a processing means 5 for storing a test program and a field image such as a test field image, feeding image signals for this stored test field, etc., to said display means 3 while detecting the pointing provided by said pointing means 4, and executing the process for computing the test results on the basis of (1) the detection results and (2) the stored test program, and a notifying means 6 for notifying the testee the test results provided by the processing means 5.

The display means 3 comprises a matrix screen 2 made of liquid crystal, and an electric circuit (not shown) that receives image signals fed in and applies a predetermined voltage successively to X and Y electrodes disposed on the matrix screen 2, the liquid crystal display means itself being known so that a detailed description thereof is omitted. In addition, the image displayed by the screen 2 may be a monochrome image or a color image. However, the display means 3 is not limited to a liquid crystal device and use may be made of a plasma display, digitizer or the like.

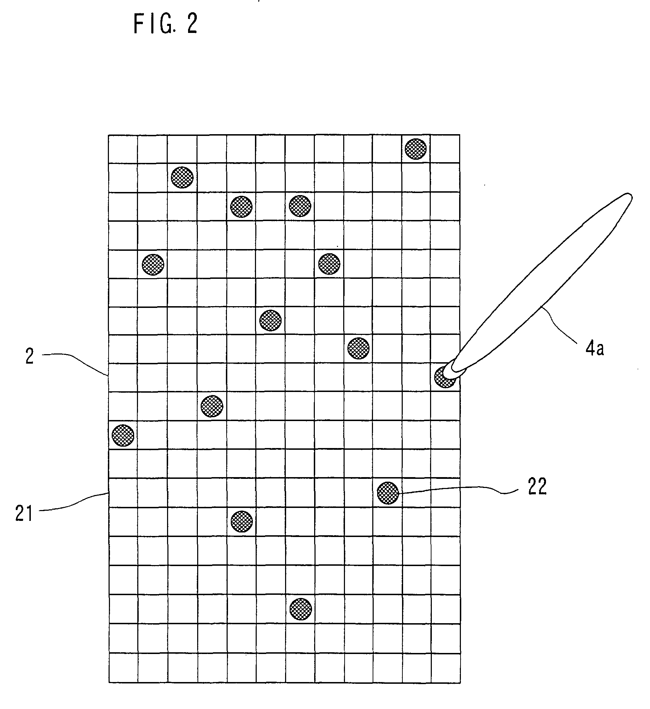

The pointing means 4 is basically a known pen type 4a shown in Fig. 2 and is used such that its tip is contacted with the screen 2 of the liquid crystal display means 3 at a particular position therein. Thereby, the liquid crystal display means 3 displays, by an electromagnetic or optical function or any one of various other physical sensing functions, the pen point contact position at a predetermined location within the test field displayed on the screen 2.

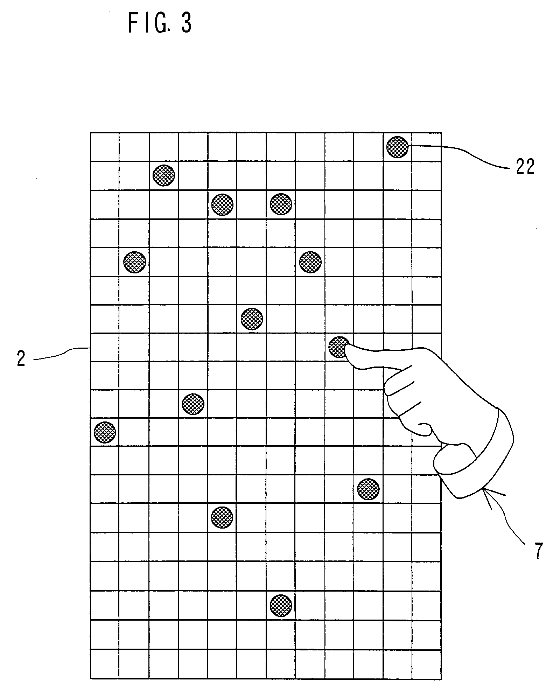

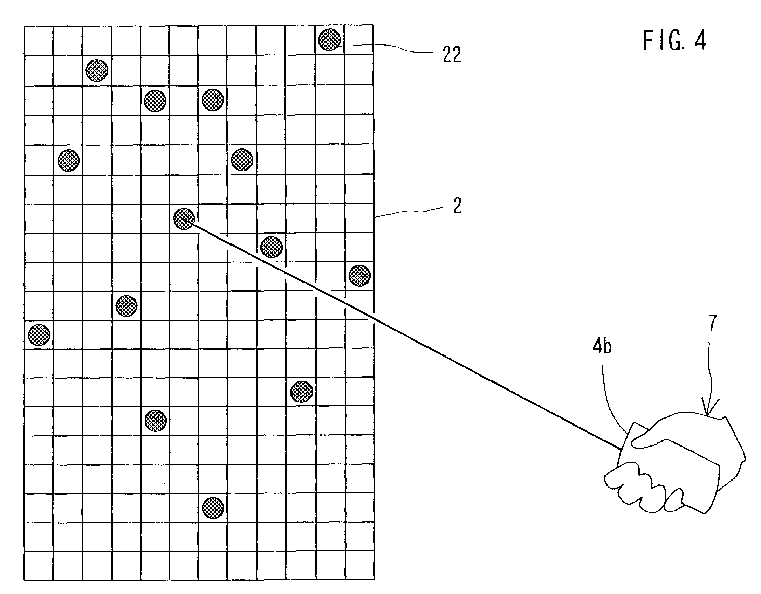

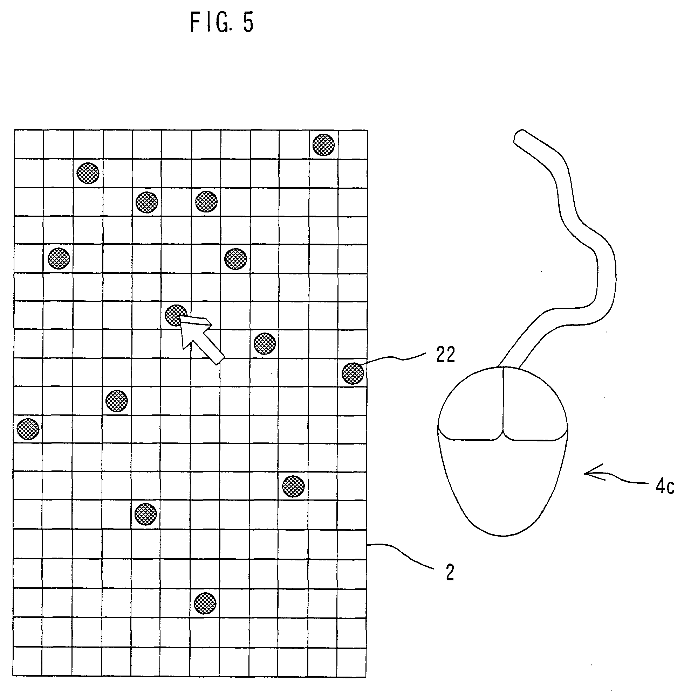

As for the indicating means 4, various means that follow may be used. Fig. 3 shows such means wherein the screen 2 of the display means is formed as a touch screen to allow a testee 7 to press his finger thereagainst to put a dot in a square; Fig. 4 shows such means wherein the testee 7 grips a laser pointer 4b to emit a laser beam to a desired square on the screen 2 so as to make an entry of a dot or line; and Fig. 5 shows such means wherein an entry is made with a mouse 4c, these means being employed according to the testee's finger function, etc.

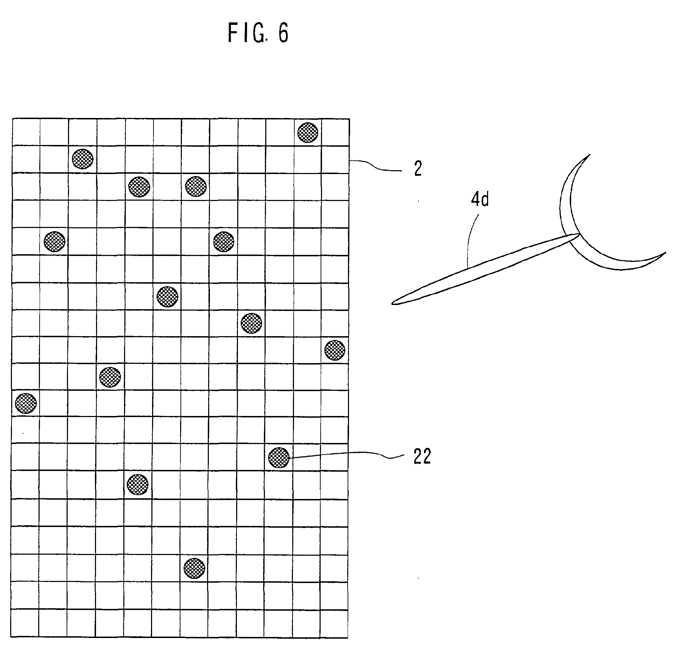

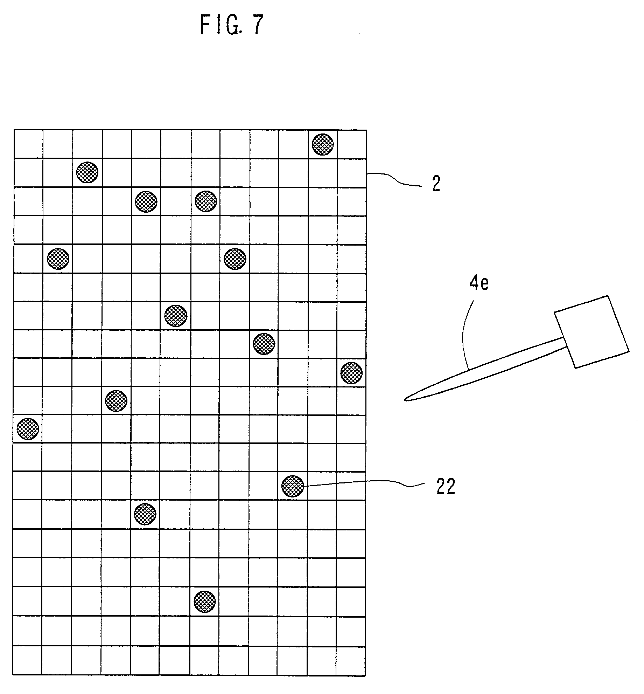

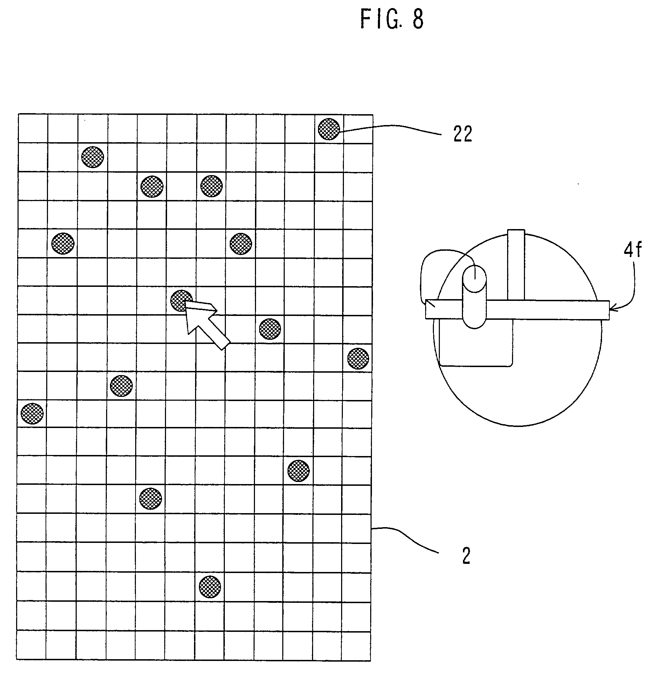

Fig. 6 shows a head stick 4d for use by a hand finger-functionally handicapped testee, and Fig. 7 shows a mouth stick 4e for a similar purpose, the point of such sticks exerting a mechanical or electromagnetic action on a square contacted thereby according to the sensing function of the entry screen 2. Fig. 8 shows a head master 4f for use by a testee who is limited not only in hand finger movement but also limited in the angle through which, or force with which, he can turn his head, designed to have a function similar to that of a mouse, making it possible for a moderate turning movement of the head to point a desired square in the screen 2.

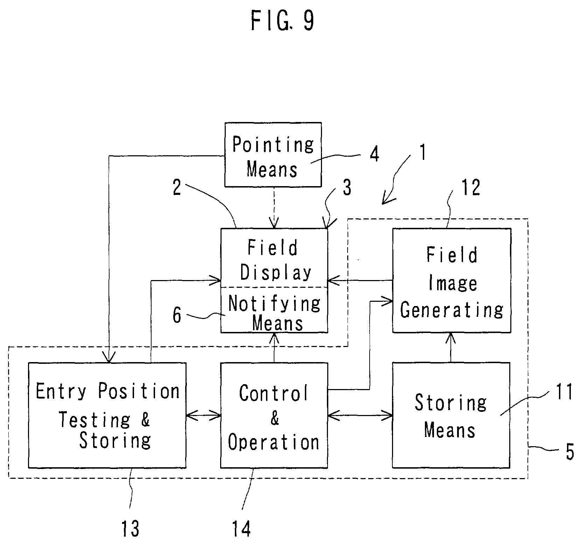

The processing means 5 in this example, as shown in Fig. 9, comprises a storing means 11 for storing in advance a test program and field images of a test field, etc., a field image generating means 12 for generating image signals for the test field, etc., on the basis of the test program stored in the storing means 11, and feeding them to the liquid crystal display means 3, an entry position testing and storing means 13 for detecting pointed position provided by the pointing means 4, and a control and operation means 14 for computing the test results on the basis of (1) the detection results provided by said means 13 and (2) the test program read from the storage means 11.
Particularly, the storage means 11 in this example stores a test program, as shown in Fig. 2, comprising the steps of displaying as a test field image a plurality of squares 21 on the screen 2 of the liquid crystal display means 3, and computing the test results from the number and positions of squares 21 entry-pointed by the pointing means 4 and from the time taken to complete indication. Upon execution of this test program in the storage means 11, the field image generating means 12 generates image signals for the squares 21 and then feeds them to the liquid crystal display means 3. The liquid crystal display means 3 displays the squares 21 on the screen 2 in response to the image signals for the squares 21 fed from the image generating means 12. The positions of the squares 21 entry-pointed by the pointing means 4 manipulated by the testee are detected by the detecting and storing means 13, and the control and operation means 14 computes the test results on the basis of the pointed positions of the squares 21 provided by the detecting and storing means 13, and of the test program read from the storage means 11.

In the case of a random dotting system, prior to the execution of this test program, the testee is told by an instructor or the like to indicate a predetermined number of optional squares 21 by the indicating means 4 with the intension of optionally and uniformly distributing them. The control and operation means 14 computes the test results from the indicated portions of a predetermined number of squares 21 and from an evaluation criterion set in the test program. This evaluation criterion can be set on the basis of a thesis given on pages 283 through 290, Vol. 14 of Japanese Government Pension Hospital Yearly Report, etc. The evaluation criterion, in outline, is such that the evaluation is determined on the basis of the distribution of pointed positions and the time taken to point individual positions and the time taken to complete the dotting; more particularly, the more the distribution of pointed positions is varied and the shorter the time taken to point individual positions and to complete the dotting, the higher the value, that is, an evaluation of the degree of intelligence lowering being slightness is given. In addition, it is desirable that the squares 21 pointed by the pointing means 4 be shown by dot impressions in the form of hatched circles 22 for recognition. It may be arranged that the display of the hatched circles 22 for recognition disappears immediately after pointing or remains until the test is completed.

The notifying means 6 in this example is disposed adjacent the screen 2 of the liquid crystal display means 3 or borrows part of the screen 2 to display thereon the test results computed by the operating means 14 to thereby notify the testee of the same. In addition, as for the notifying means, such arrangement for making notification by liquid crystal screen display may be replaced by an arrangement for making notification, for example, by printing the test results on a printer or by means of sound. As for the form of display of the test results, it is, for example, level 1, level 2, and so on.

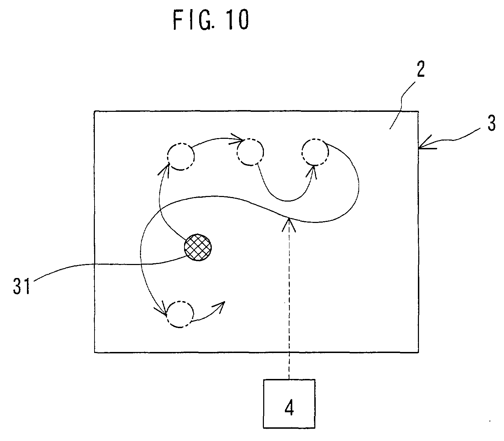

In this connection, the storage means 11 may contain a test program, as shown in Fig. 10, comprising the steps of displaying as an in-field image a target 31 to be chased that is moving at random as denotedby arrowlines on the screen 2 of the liquid crystal display means 3 for a given time, and computing the test results that consist of the time or distance passed upon the target 31 having been successfully pointed by the pointing means 4 and the time or distance passed upon the target 31 having been unsuccessfully pointed by the pointing means 4. In this example, therefore, upon execution of the test program in the storage means 11, the image generating means 12 feeds image signals to the liquid crystal display means 3 and the latter displays the target 31 moving at random on the screen 2 according to the image signals fed in. The position of the target 31 successfully pointed by the pointing means 4 manipulated by the testee is detected by the detecting and storing means 13, and the controlling and operating means 14 computes the test results on the basis of (1) the successfully indicated position of the target 31 provided by the detecting and storing means 13 and (2) the test program stored in the storage means 11.

In addition, prior to the execution of this test program, the testee is told by an instructor or the like to correctly chase and point the target 31 moving at random by the pointing means 4. The controlling and operating means 14 computes the test results on the basis of (1) the time or distance passed upon the target 31 having been successfully indicated by the pointing means 4 and the time or distance passed upon the target 31 having been unsuccessfully pointed by the indicating means 4 and (2) the evaluation criterion set in the test program. The evaluation criterion, in outline, is such that the longer the time or distance passed upon the target 31 having been successfully pointed by the pointing means 4, the higher the value, that is, an evaluation of the degree of intelligence lowering being slightness is given. In this example also, the notifying means, borrowing part of the screen 2 of the liquid crystal indicating means 3, notifies the testee of the test results, for example, in terms of level 1, level 2, and so on.

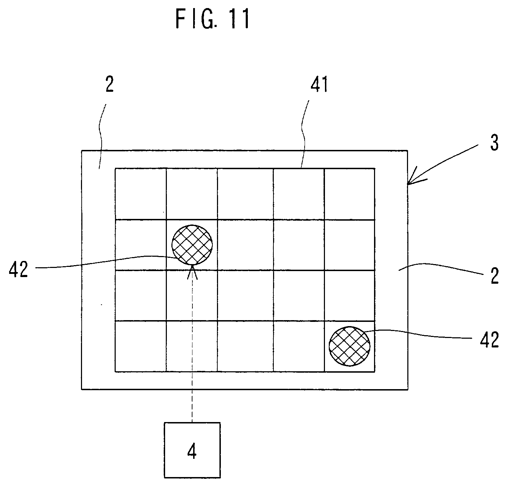

Further the storage program contained in the storage means 11 may includes a test object program, as shown in Fig. 11, comprising the steps of displaying a plurality of squares 41 on the screen 2 of the liquid crystal display means 3 as a test object image, displaying for a given time the target 42 in one of the randomly successively selected squares 41 from the thus displayed squares 41, and computing the test results from the number of targets 42 successfully pointed by the pointing means 4 during the display of the targets 42 and the number of targets 42 displayed during this test.

In this example, therefore, upon execution of the test program in the storage means 11, the image generating means 12 generates image signals for a plurality of squares 41 and for the target 42 to be displayed in one of the squares 41 randomly successively selected from said plurality of squares, and feeds them to the liquid crystal display means 3 and the latter displays these squares 41 on the screen 2, and also displays the target 42 in one of the randomly successively selected squares 41 for a given time. The position of the target 42 successfully pointed by the pointing means 14 manipulated by the testee is detected by the detecting and storing means 13, and the controlling and operating means 14 computes the test results on the basis of (1) the pointed position of the target 42 provided by the detecting and storing means 13 and (2) the test program stored in the storage means 11.

In this case, prior to the execution of this test program, the testee is told by an instructor or the like to successively successfully point the targets 42 displayed in the squares 41 such that each pointing can last even for an instant, for example. The controlling and operating means 14 computes the test results from the number of times the target 42 is successfully pointed by the pointing means 4 and from the evaluation criterion set in the test program. The evaluation criterion, in outline, is such that the larger the number of times the target 42 is successfully pointed by the pointing means 4 during testing, the higher the value, that is, an evaluation of the degree of intelligence lowering being slightness is given. In this example also, the notifying means 6, borrowing part of the screen 2 of the liquid crystal pointing means 3, displays the test results and notifies them, for example, in terms of level 1, level 2, and so on.

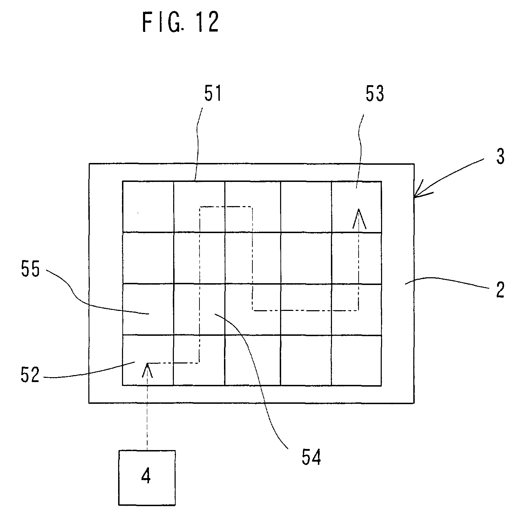

Further, the storage means 11 may store a test program, as shown in Fig. 12, comprising the steps of displaying a plurality of squares 51 on the screen 2 of the liquid crystal display means 3 as a test field image, making a comparison between the order of pointing of the squares 51 pointed by the pointing means 4 and the preset order of routing the squares (shown in chained lines in Fig. 12) to be pointed, and computing the test results from the time taken for the squares 51 to be pointed in the order of routing of the squares to be pointed that is preset in the test program or from the number of times of erroneous indication.

In this example, therefore, upon execution of this test program, the image generating means 12 generates image signals for a plurality of squares 51 and feeds them to the liquid crystal display means 3, and the latter displays the squares 51 on the screen 2. The positions of the squares 51 pointed by the pointing means 4 manipulated by the testee are detected by the detecting and storing means 13, and the controlling and operating means 14 computes the test results on the basis of (1) the pointed positions of the squares 51 provided by the detecting and storing means 13 and (2) the test program.

In this case, if a comparison between the order of indication of the squares 51 actually pointed by the pointing means 4 and the setting order of the squares to be pointed shows a difference therebetween, for example, if a square 55 deviating from a preset square 54 is pointed, the information to this effect can be displayed on the screen 2. Such error display arrangement may be replaced by an arrangement for making notification by means of sound. Prior to the execution of this test program, the testee is told by an instructor or the like, for example, to point some squares among a plurality of squares 51 successively from a start square 52 to a goal square 53.

The testee is not told of the order of arrangement of the squares to be pointed that is preset in the test program or may be given a suggestion, for example, that the set order is such as to move in zigzags from the lower left to the upper right. The controlling and operating means 14 computes the test results from the time taken for the squares 51 to be pointed in the preset order or the number of times of erroneous indication, and the evaluation criterion set in the test program. The evaluation criterion, in outline, is such that the shorter the time taken for the squares 51 to be pointed in the preset order of routing of the squares to be pointed or the fewer the number of times of erroneous pointing, the higher the value, that is, an evaluation of the degree of intelligence lowering being slightness is given. In this example also, the notifying means 6, borrowing part of the screen 2 of the liquid crystal pointing means 3, notifies the testee of the test results, for example, in terms of level 1, level 2, and so on.

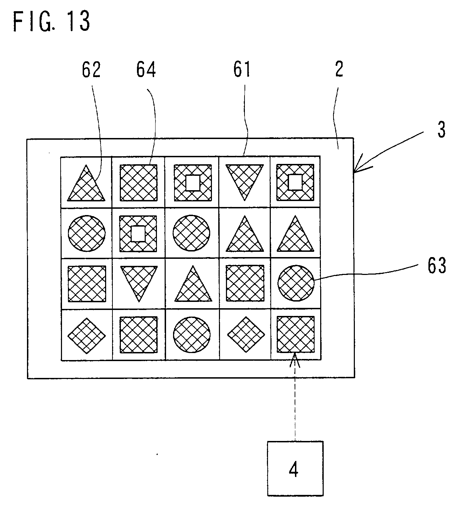

Further, the storage means 11 may store a test program, as shown in Fig. 13, comprising the steps of displaying as a test field image a plurality of different figures including triangles 62, circles 63, quadrangles 64 and etc., respectively placed in a plurality of squares 61 on the screen 2 of the liquid crystal display means 3, and computing the test results from factors including the number of some specified figures such as triangles 62, circles 63 or quadrangles 64 pointed by the pointing means 4. In this example, therefore, upon execution of this test program, the image generating means 12 generates image signals for the squares 61 and for figures including triangles 62, circles 63, quadrangles 64 and etc. to be displayed respectively in the squares 61 and feeds them to the liquid crystal display means 3 and the latter displays the squares 61 and figures including triangles 62, circles 63 and quadrangles 64 on the screen 2 on the basis of such image signals.

The positions of the squares 61 pointed by the pointing means 4 manipulated by the testee are detected by the detecting and storing means 13, and the controlling and operating means 14 computes the test results on the basis of (1) the pointed positions of the squares 61 provided by the detecting and storing means 13 and (2) the test program stored in the storage means 11. In addition, in this case, prior to the execution of this test program, the testee is told by an instructor or the like to successively point (select), for example, the same figures of one type and then the same figures of another type upon completion of pointing of said same figures of one type. In addition, when the pointing of the same figures of one or another type has been completed, or when a different figure is erroneously pointed during the pointing of the same figures of one or another type, such information may be displayed on the screen 2.

Such arrangement for displaying testees pointing operation completion and errors may be replaced by an arrangement for notifying the testee of the results by means of sound or the like as described above. The controlling and operating means 14 computes the test results from the time taken to complete all pointing of the figures, the number of times of erroneous indication, and the evaluation criterion set in the test program. The evaluation criterion, in outline, is such that the shorter the time taken to complete all pointing of the figures and the smaller the number of times of erroneous pointing, the higher the value, that is, an evaluation of the degree of intelligence lowering being slightness is given. In this example also, the notifying means 6, borrowing part of the screen 2 of the liquid crystal display means 3, notifies the testee of the test results, for example, in terms of level 1, level 2, and so on.

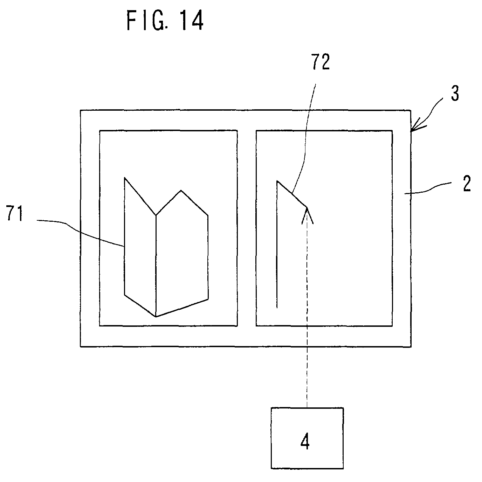

Further, the storage means 11 may store a test program, as shown in Fig. 14, comprising the steps of displaying as test field images a figure 71 in part of the field (in this case, the left half) on the screen 2 of the liquid crystal display means 3, and computing the test results from differences between a locus 72 pointed for replicating purposes in the other portion of the screen 2 (in this case, the right half) by the pointing means 4 and the figure 71 previously displayed. In this example, therefore, upon execution of this test program, the image generating means 12 generates image signals for the figure 71 and feeds them to the liquid crystal display means 3, and the latter displays the figure 71 on the screen 2 on the basis of the image signals fed from the image generating means 12.

The position of the pointing locus 72 drawn by the pointing means 4 manipulated by the testee is detected by the detecting and storing means 13, and the controlling and operating means 14 computes the test results on the basis of (1) the indication locus 72 detected by the detecting and storing means 13 and (2) the test program stored in the storage means 11. In addition, in this case, the pointing locus 72 is also supposed to remain displayed on the screen 2; however, it may not be displayed from the beginning or may be displayed only for a short time, according to the intelligence level of the testee. As for the figure 71 to be displayed on the screen 2, it is desirable that various shapes can be selected. Prior to the execution of the test program, the testee is told by an instructor or the like to draw the same figure as the figure 71 displayed on the screen 2, in the blank of the screen 2 by means of the pointing means 4. The controlling and operating means 14 computes the test results from the pointing locus 72 detected by the detecting and storing means 13, that is, the figure drawn by the testee, and the evaluation criterion set in the test program. The evaluation criterion, in outline, is such that the smaller the shift between the preset display position of the figure 71 and the corresponding position of the pointing locus 72, the higher the value, that is, an evaluation of the degree of intelligence lowering being slightness is given. In this example also, the notifying means 6, borrowing part of the screen 2 of the liquid crystal display means 3, notifies the testee of the test results, for example, in terms of level 1, level 2, and so on.

In addition, what has been described so far shows examples of intelligence test systems; however, the same may be utilized to provide intelligence recovery training systems.

As has been described so far, the present invention has a great deal of usefulness in that intelligence test and intelligence recovery training can be readily effected and the test results can be known at once.

## Claims

1. An intelligence test and recovery training system, comprising;
(a) a test program and field image storing means for storing at least one test program, and a field image that consists of a test field constructed according to said program and a guide pattern used for recognition, chase, replicating or the like and that is selectively explicitly shown in said field,
(b) a field image generating means for reading the field image from said storage means according to the program and generating image signals,
(c) a field image displaying means having a screen frame for receiving said image signals and displaying the field image,
(d) pointing means that, in order to allow a testee or subject to make an entry of a dot or line in the test field displayed in said screen frame, on the basis of a system planned in a test program therefor, is used to point the entry position therefor,
(e) an entry position generating and storing means for driving said display means so as to detect the positions and locus of the dots or lines entered by said testee and selectively display the same in said field according to the program and storing the positions and locus of the dots or lines together with the lapse of time, and
(f) a controlling and operating means for evaluating the degree of intelligence or the state of intelligence recovery by computing (1) the relationship between the positions and locus of the dots or lines entered by said testee and the lapse of time, and (2) the distribution, according to said test program.

2. A system as set forth in Claim 1, wherein the controlling and operating means has a function of causing said display means to display the results of evaluation of the degree of intelligence or the state of intelligence recovery.

3. A system as set forth in Claim 1 or 2, wherein the test field includes a plurality of visible squares arranged in matrix form and the coordinates of dots or lines are specified square by square.

4. A system as set forth in Claim 3, wherein the testee's dotting or line drawing system planned in said test program is a random dotting system, and the computations in accord with said test program are for statistically evaluating the degree of intelligence or the state of intelligence recovery from the uniformity of distribution of dots, and the amount of movement and the amount of time, etc. involved in dotting operation.

5. A system as set forth in any one of Claims 1 through 3, wherein the testees dotting or line drawing system planned in said test program follows or replicates a guide pattern explicitly shown in the field or suggested in advance, and the computations in accord with said test program are for evaluating the degree of intelligence or the state of intelligence recovery by computing the degree of agreement between the entry positions and the guide pattern or a time lag and positional shift.
